# EUROPEAN PATENT SPECIFICATION

(11) **EP 1 949 930 B1**
(45) Date of publication and mention of the grant of the patent: **18.08.2010**
(21) Application number: 07254095.8
(22) Date of filing: 16.10.2007
(51) Int. Cl.: A61M 16/08

(54) **Medical apparatus having a connecting point for fluid**
Medizinische Vorrichtung mit Anschlusspunkt für Fluid
Appareil médical doté d'un point de raccordement de fluides

(30) Priority: 24.01.2007 DE 102007003594
(43) Date of publication of application: 30.07.2008
(73) Proprietor: Dräger Medical AG & Co. KG, 23558 Lübeck (DE)
(72) Inventor: Schermeier, Olaf, 23560 Lübeck (DE); Wotha, Gerd, 23626 Warnstorf (DE); Steeger, Markus, 23564 Lübeck (DE); Tappehorn, Ludger, 23617 Stockelsdorf (DE)
(74) Representative: Greenwood, John David

(56) References cited:
- EP-A- 1 731 089
- WO-A-2006/134428
- US-A- 5 910 776
- US-A1- 2007 222 603

## Description

The present invention relates to a medical apparatus having a connecting point for fluid, a hose, and a nozzle which can be connected on the one hand to the connecting point for fluid and on the other hand to the hose to be a position in rotation which is freely selectable in the given case, to make a connection for fluid between the connecting point for fluid and the hose, the hose being provided with a transponder at its end for connection to the nozzle, and having a read/write device for communicating with the transponder, which read/ write device has an aerial in the region of the connecting point for fluid.

A typical example of a medical apparatus of this kind is a ventilation apparatus to whose connecting point for ventilating gas a hose, which feeds into the ventilation apparatus, can be connected via a nozzle. Hoses of this kind are provided at their end adjacent the ventilation apparatus with transponders which enable the ventilation apparatus, which has an associated read/write device, to exchange information with the transponder and by this means to supply information on the nature and other properties of the hose which is connected on. The aerial of the read/write device is situated close to the connecting point for ventilating gas of the ventilation apparatus; the aerial may for example surround the said connecting point for ventilating gas concentrically.

A problem with apparatus of this kind is that there is inevitably a certain distance between the aerial on the connecting point for fluid and the transponder on the hose because the nozzle is situated between the hose and the connecting point for fluid. Because the range is limited when it is radio-frequency signals which are exchanged between the aerial of the read/write device and that of the transponder, problems may arise in the transmission of the signals. A further problem is that the connections of the nozzle to the connecting point for fluid on the one hand and to the hose on the other hand do not preset fixed or preset positions in rotation for the connecting point for fluid and the nozzle on the one hand and for the nozzle and the hose on the other hand, i.e. the connections can, in principle, be made with the components in any desired position relative to one another in rotation. What this means is that both the position of the nozzle in rotation relative to the connecting point for fluid may vary over a relative angle in rotation of 360° and so also may the relative position in rotation of the nozzle in relation to the hose. Due to these circumstances, it cannot be foreseen what position the aerial of the transponder will be in relative to the aerial of the read/write device.

What is more, the rotatable connection of both the connecting point for fluid and the hose to the nozzle stops there from being, for any practical point of view, an electrically conductive connection, because a connection of this kind could only be obtained at considerable structural cost and complication, such for example as by means of sliding contacts.

EP-A-1731089 describes a multi-part medical system which includes at least two components connectable via position-determining connection means whereby at least one component is intended to remain close to the patient in the connected and in the unconnected state of the system and at least one component can be removed or replaced by other components in the unconnected state, whereby the component intended to remain close to the patient contains means for data storage which can be written or read out via an interface which is mechanically integrated into the position-determining connection means.

WO-A-2006134428 describes an intelligent antenna system for extending the effective communication range of a machine-readable tag. The system includes intelligence that allows one of a plurality of extension antennas to be active at any given time in order to both facilitate communications and safeguard the system. In a further embodiment of the invention, the machine-readable tag includes combined RFID and NFC functionality where both RFID listening functions and NFC read-write functions are to be available in the same NFC communications logic. The NFC communications logic chip is selectively connected to a first antenna coil for RFID operations and a second for NFC operations. It is an object of the present invention to improve a medical apparatus having a connection for a hose which is made via a nozzle in such a way that it is ensured that signals will be transmitted with great reliability between the read/write device of the medical apparatus and the transponder on the hose.

The present invention is as claimed in the claims.

Provision is made in accordance with the invention for the nozzle to be provided, in the region of each of its ends for connection, with a coil, the axis of each coil being substantially co-axial with the axis of rotation about which the relative position in rotation of the nozzle in relation to the connecting point for fluid and the hose is variable in the respective cases. The coils are connected together via an electrical conductor, a capacitor being connected to the conductor and the coils as a further circuit component to form an electrical oscillating circuit to transmit signals between the read/write device and the transponder. The electrical oscillating circuit forms an inductive bridging element which spans a large part of the distance between the aerial of the read/write device and that of the transponder, which affords a considerable improvement in signal transmission over pure radio frequency radio transmission. What is more, the arrangement of the coils to be symmetrical in rotation with the regions of the ends for connection of the nozzle ensures that a field is generated which is substantially symmetrical in rotation, which means that signal transmission is not dependent on the relative position in rotation of the nozzle in relation to the connecting point for fluid on the one hand and in relation to the hose on the other hand. The coil comprises at least one turn of wire whose centre axis coincides with the axis of rotation about which the position in rotation of the connection of the nozzle to the connecting point for fluid or to the hose is variable. The coil may have a plurality of turns which extend in the form of a helix having an axis which coincides with the said axis of rotation. Alternatively, the coil may also be in the form of a spiral whose centre point lies on the said axis of rotation and which extends in a plane perpendicular to the axis of rotation.

The coils act in this case both as aerials and also as inductive elements of the oscillating circuit. By choosing a suitable capacitance for the capacitor which is connected in, and if required a suitable impedance for a resistor which is connected in, the resonant frequency and the width of the resonance curve can be preset in a suitable way so that they are matched to the transmitting and receiving frequency of the read/write device and the transponder.

The coil which is situated at the end for connecting the nozzle to the connecting point for fluid receives a radio-frequency signal which is emitted by the aerial of the read/write device and which makes its way, via the oscillating circuit, to the coil situated at the other end for connection, a process which at that point results in a corresponding radio-frequency signal being emitted which is received by the aerial of the transponder. The other way round, a radio-frequency signal emitted by the aerial of the transponder makes its way via the oscillating circuit to the coil situated at the end of the nozzle adjacent to the connecting point for fluid and there causes a corresponding radio-frequency signal to be emitted which is received by the aerial of the read/write device.

Provision may be made for a core of ferromagnetic material to be introduced into the space within at least one coil to cause the field produced by the coil to be focussed. The core may be a separate component in this case. Alternative, the nozzle or parts thereof may be constructed from ferromagnetic material.

The invention is explained below by reference to exemplary embodiments shown in the drawings, in which:
Fig. 1 is a schematic view of a ventilation apparatus having a hose connected thereto via a nozzle;
Fig. 2 is a schematic view of the components which are used to construct a nozzle to be used in connection with the present invention;
Fig. 3 is a perspective view of a nozzle to be used in connection with the present invention.

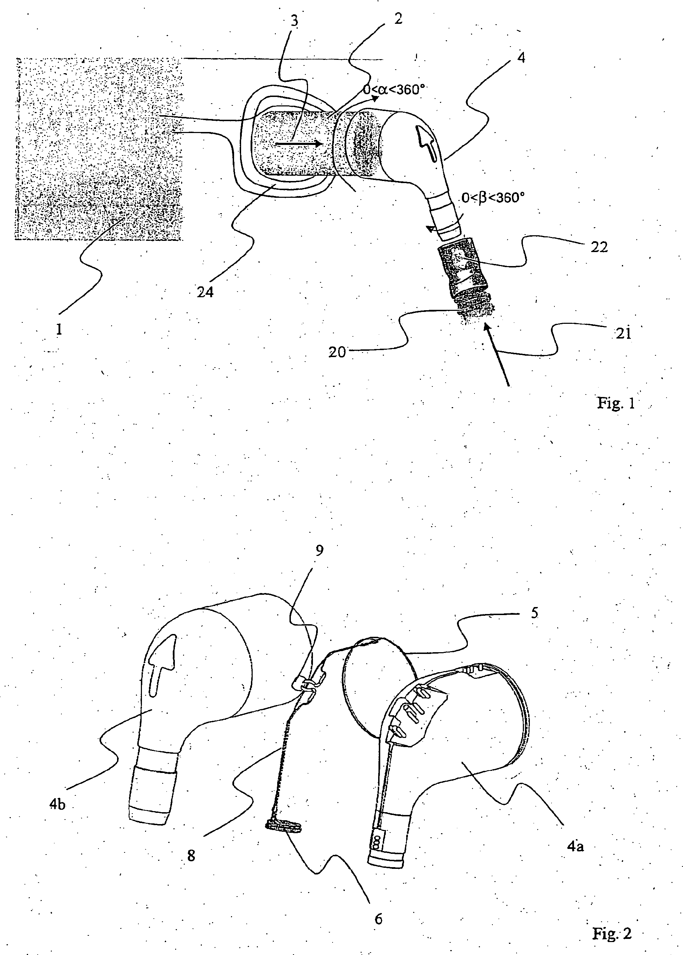

Fig. 1 is a schematic view of part of a medical apparatus, namely a medical ventilation apparatus, the ventilation apparatus proper being indicated by the reference numeral 1. Able to be connected to the connecting point for fluid 2 of the ventilation apparatus 1 is a nozzle 4. The connection is made by a push-on connection, which means that the relative position in rotation α of the connecting point for fluid 2 and the nozzle 4 in relation to one another is totally variable: α = 0 - 360°. Able to be connected to the opposite end of the nozzle 4 is the hose 20, likewise by means of a push-on connection, which means that the relative angle in rotation β between the hose 20 and the nozzle 4 is also totally variable. At its end for connection, close to the nozzle 4, the hose 20 is provided with an RFID transponder 22, in which information on the type and other properties of the hose 20 may be stored.

This information is read by a read/write device associated with the ventilation apparatus 1, which read/write device has an aerial 24 which is arranged in a position surrounding the connecting point for fluid 2. The RFID transponder 22 on the hose responds to an enquiry signal from the read/write device which is emitted via the aerial 24 with response signals which are finally picked up in turn by the aerial 24 of the read/write device.

To ensure that data is transmitted reliably between the aerial 24 of the read/write device and the transponder 22 in spite of the distance between them caused by the nozzle 4 and in spite of the variable positions in rotation α, β of the nozzle 4 relative to the respective items connected to it, provision is made for a coil to be arranged in the region of each end for connection of the nozzle 4, these coils being denoted by reference numerals 5 and 6 in Fig. 2. The coils are connected together by at least one electrical conductor 8, with a capacitor 9 also being connected in as a further circuit component. The electrical properties of the coils 5, 6, the conductor 8 and the capacitor 9 are selected in such a way that the resonant frequency of the oscillating circuit which is formed is matched to the transmitting and receiving frequency of the read/write device and the transponder 22.

Each coil 5, 6 is so arranged in the associated end for connection of the nozzle 4 that its axis coincides with the axis of rotation about which the relative position in rotation of the given end for connection of the nozzle is variable. The axis about which the position in rotation of the connection of the nozzle 4 to the connecting point for fluid 2 is variable is indicated schematically by an arrow 3. In a similar way, the axis about which the relative position in rotation of the hose in relation to the opposite end of the nozzle 4 is variable is indicated by an arrow 21.

Because the coils 5, 6 are set up to be symmetrical in rotation with the respective connections of the nozzle 4, transmission which is not dependent on the positions in rotation of the nozzle 4 relative to the connecting point for fluid 2 and to the flexible hose 20 is ensured. Also, because the signals are conveyed via the oscillating circuit 5, 6, 8, 9, considerably better signal transmission is obtained in comparison with pure radio-frequency radio transmission.

Fig. 2 shows a first embodiment of nozzle to be used in connection with the present invention, there being a first inner wall part 4a which is formed, at each of the ends for connection, with sunken regions in which the coils 5, 6 are accommodated. The coils 6 and 5 are wound from wire which joins up with the conductor 8, which is in the form of a wire conductor and into which at least one capacitor 9 is connected as a circuit component between the coils. The oscillating circuit 5, 6, 8, 9 having been fitted to the inner part 4a of the nozzle 4, a second, outer, housing part 4b can be slid over, thus embedding the oscillating circuit between the inner and outer walls of the nozzle 4. This embedding within the housing is advantageous because the nozzle 4 can then be cleaned and disinfected in the usual way.

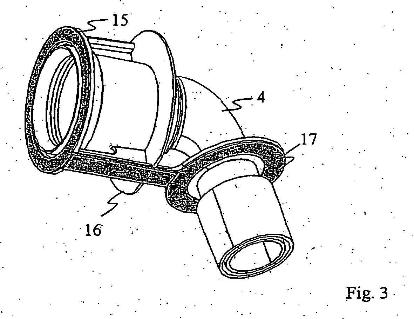

Fig. 3 shows an alternative embodiment of nozzle 4 to be used with the invention. In this case the housing part of the nozzle 4 is provided with annular surfaces 15 and 17, with the plane of the annulus being in each case perpendicular to the axis of rotation about which the connection of the nozzle 4 to the item to which it is connected is variable. To put it another way, the planes of the annular bodies 15, 17 lie substantially parallel to the plane of the associated openings for connection of the nozzle 4. As is shown by the example of the annular surface 17, the said annular body may in this case be at a certain distance from the plane of the opening for connection.

The two annular bodies 15, 17 are connected by a bridge of material 16. Also provided is a flexible printed-circuit board which has two annular regions corresponding to the annular surfaces 15 and 16, which annular regions are connected together by a region in bridge form corresponding to the bridge of material 16. Mounted on this flexible printed-circuit board in the annular regions are the coils 5 and 6 (not shown in Fig. 3), with the electrical connecting conductor and the capacitor being mounted on that part of the printed-circuit board which is in bridge form and which comes to bear against the bridge of material 16. The flexible printed-circuit board may be provided with adhesive on one side, to enable it to be adhesive-bonded in this way to the annular surfaces 15, 17 and the bridge of material 16 situated between them.

In this embodiment, the coil to be arranged on the annular body 15 may for example be in the form of a spiral having a plurality of turns across the annular surface of the annular body 15.

## Claims

1. Medical apparatus having a connecting point for fluid (2), a hose (20), and a nozzle (4) which can be connected on the one hand to the connecting point for fluid (2) and on the other hand to the hose (20) to be in a position in rotation which is freely selectable in the given case, to make a connection for fluid between the connecting point for fluid (2) and the hose (20), the hose (20) being provided with a transponder (22) at its end for connection to the nozzle (4), and having a read/write device for communicating with the transponder (22), which read/write device has an aerial (24) in the region of the connecting point for fluid (2), **characterised in that** the nozzle (4) is provided, in the region of each of its two ends for connection, with a coil (5, 6), the axis of each coil being substantially co-axial with the axis of rotation about which the relative position in rotation of the nozzle (4) is variable, and **in that** the two coils (5, 6) are connected by electrical conductors (8) to which at least one capacitor (9) is connected as a circuit component, the coils (5, 6), electrical conductors (8) and circuit component(s) (9) forming an electrical oscillating circuit to transmit signals from the read/write device to the transponder (22) and vice versa.

2. Medical apparatus according to claim 1, in which the turns of each coil (5, 6) extend round the flow passage of the nozzle (4) in the region of the associated openings for connection.

3. Medical apparatus according to claim 2, in which the coils (5, 6) are embedded within the wall of the nozzle (4) in the region of respective ones of the openings for connection.

4. Medical apparatus according to claim 3, in which the electrical conductors (8) and the circuit components (9) of the oscillating circuit are also embedded within the wall of the nozzle.

5. Medical apparatus according to claim 1, in which the nozzle (4) is provided in the region of each of its ends for connection with an annular surface (15, 17) which is arranged to extend concentrically around the outer wall of the nozzle (4), the annular surfaces (15, 17) being connected together by an elongated bridge of material (16), and in that the coils (5, 6), the electrical conductors (8) and the circuit component (9) of the oscillating circuit are mounted on a flexible printed-circuit board which is adhesive-bonded to the annular surfaces (15, 1 7) and the bridge of material (16), thus causing each of the coils to rest against a respective one of the annular surfaces.

6. Medical apparatus according to claim 5, in which the annular surfaces (15, 17) and the bridge of material (16) are injection moulded from plastics material in one piece with the nozzle (4).

7. Medical apparatus according to one of the foregoing claims, the space within at least one coil (5, 6) includes a core of ferromagnetic material so as to cause the field produced by the coil to be focussed.

## Patentansprüche

1. Medizinische Vorrichtung mit einem Fluid-Anschlusspunkt (2), einem Schlauch (20) und einer Tülle (4), die einerseits mit dem Fluid-Anschlusspunkt (2) und andererseits mit dem Schlauch (20) mit jeweils frei wählbarer Drehstellung verbindbar ist, um eine Fluid-Verbindung zwischen dem Fluid-Anschlusspunkt (2) und dem Schlauch (20) herzustellen, wobei der Schlauch (20) mit einem Transponder (23) an seinem Anschlussende an die Tülle (4) versehen ist, und mit einem Schreiblesegerät zur Kommunikation mit dem Transponder (22), welches Schreiblesegerät eine Antenne (24) im Bereich des Fluid-Anspruchspunktes (2) aufweist, **dadurch gekennzeichnet, dass** die Tülle (4) im Bereich jedes ihrer beiden Anschlussenden mit einer Spule (5,6) versehen ist, wobei die Achse jeder Spule im Wesentlichen koaxial zu der Drehachse verläuft, um die die relative Drehstellung der Tülle (4) variabel ist, und dass die beiden Spulen (5, 6) durch elektrische Leitungen (8) verbunden sind, an die zumindest ein Kondensator (9) als Schaltungskomponente angeschlossen ist, wobei die Spulen (5, 6), elektrische Leitungen (8) und Schaltungskomponente(n) (9) einen elektrischen Schwingkreis zur Übertragung von Signalen vom Schreiblesegerät zum Transponder (22) und umgekehrt bilden.

2. Medizinische Vorrichtung nach Anspruch 1, bei der die Windungen jeder Spule (5, 6) um den Strömungskanal der Tülle (4) den Bereich der zugehörigen Anschlussöffnungen umlaufen.

3. Medizinische Vorrichtung nach Anspruch 2, bei der die Spulen (5, 6) in die Wand der Tülle (4) jeweils im Bereich der Anschlussöffnungen eingebettet sind.

4. Medizinische Vorrichtung nach Anspruch 3, bei der die elektrischen Leitungen (8) und die Schaltungskomponenten (9) des Schwingkreises ebenfalls in die Wand der Tülle eingebettet sind.

5. Medizinische Vorrichtung nach Anspruch 1, bei der die Tülle (4) im Bereich jedes ihrer Anschlussenden mit einer Ringfläche (15, 17) versehen ist, die konzentrisch um die Außenwand der Tülle (4) umlaufend angeordnet ist, wobei die Ringflächen (15, 17) durch eine längliche Materialbrücke (16) miteinander verbunden sind, und die Spulen (5, 6), die elektrischen Leitungen (8) und die Schaltungskomponente (9) des Schwingkreises auf einer flexiblen Leiterplatte angebracht sind, die auf den Ringflächen (15, 17) und der Materialbrücke (16) aufgeklebt ist, so dass jede der Spulen an einer jeweiligen der Ringflächen anliegt.

6. Medizinische Vorrichtung nach Anspruch 5, bei der die Ringflächen (15, 17) und die Materialbrücke (16) in einem Stück mit der Tülle (4) aus Kunststoff gespritzt sind.

7. Medizinische Vorrichtung nach einem der vorhergehenden Ansprüche, bei der der Innenraum zumindest einer Spule (5, 6) einen Kern aus ferromagnetischem Werkstoff aufweist, um eine Fokussierung des von der Spule erzeugten Feldes herbeizuführen.

## Revendications

1. Appareil médical ayant un point de connexion pour un fluide (2), un tuyau (20) et une buse (4) qui peut être raccordée d'une part au point de connexion pour fluide (2) et d'autre part au tuyau (20), de façon à se trouver dans une position en rotation qui est librement sélectionnable dans le cas indiqué, afin d'obtenir une connexion pour un fluide entre le point de connexion pour fluide (2) et le tuyau (20), le tuyau (20) étant doté d'un transpondeur (22) à son extrémité pour connexion à la buse (4) et ayant un dispositif de lecture/écriture pour communiquer avec le transpondeur (22), ledit dispositif de lecture/écriture ayant une antenne (24) dans la zone du point de connexion pour fluide (2), **caractérisé en ce que** la buse (4) est dotée, dans la région de chacune de ses deux extrémités pour connexion, d'une bobine (5, 6), l'axe de chaque bobine étant sensiblement coaxial à l'axe de rotation autour duquel varie la position relative en rotation de la buse (4), et **en ce que** les deux bobines (5, 6) sont connectées par des conducteurs électriques (8) auxquels au moins un condensateur (9) est connecté comme un composant de circuit, les bobines (5, 6), les conducteurs électriques (8) et les composants de circuit (9) formant un circuit oscillant électrique pour transmettre des signaux du dispositif de lecture/écriture au transpondeur (22) et inversement.

2. Appareil médical selon la revendication 1, dans lequel les tours de chaque bobine (5, 6) s'étendent autour du passage d'écoulement de la buse (4) dans la région des ouvertures associées pour connexion.

3. Appareil médical selon la revendication 2, dans lequel les bobines (5, 6) sont intégrées dans la paroi de la buse (4) dans la région des ouvertures respectives pour connexion.

4. Appareil médical selon la revendication 3, dans lequel les conducteurs électriques (8) et les composants de circuit (9) du circuit oscillant sont également intégrés dans la paroi de la buse.

5. Appareil médical selon la revendication 1, dans lequel la buse (4) est dotée, dans la région de chacune de ses extrémités pour connexion, d'une surface annulaire (15, 17) qui est disposée de façon à s'étendre de manière concentrique autour de la paroi extérieure de la buse (4), les surfaces annulaires (15, 17) étant connectées ensemble par un pont allongé de matériau (16), et **caractérisé en ce que** les bobines (5, 6), les conducteurs électriques (8) et le composant de circuit (9) du circuit oscillant sont montés sur une carte à circuit imprimé flexible qui est collée par adhésion aux surfaces annulaires (15, 17) et au pont de matériau (16), provoquant ainsi l'appui de chacune des bobines contre une surface annulaire respective.

6. Appareil médical selon la revendication 5, dans lequel les surfaces annulaires (15, 17) et le pont de matériau (16) sont moulés par injection à partir de matière plastique en une pièce avec la buse (4).

7. Appareil médical selon l'une quelconque des revendications précédentes, l'espace dans au moins une bobine (5, 6) comprenant un noyau de matériau ferromagnétique de façon à provoquer la mise au point du champ produit par la bobine.
